# EUROPÄISCHE PATENTSCHRIFT

(11) **EP 0 919 195 B1**
(45) Veröffentlichungstag und Bekanntmachung des Hinweises auf die Patenterteilung: **18.01.2006**
(21) Anmeldenummer: 97810927.0
(22) Anmeldetag: 28.11.1997
(51) Int. Cl.: A61B 17/15

(54) **Instrumentenbaukasten für Kniegelenkprothesen**
Tool assembly for a prosthetic knee
Assemblage d'outils pour une prothèse du genou

(43) Veröffentlichungstag der Anmeldung: 02.06.1999
(73) Patentinhaber: Zimmer GmbH, 8404 Winterthur (CH)
(72) Erfinder: Stegmüller, Nicolas, 2504 Bienne (CH); Wanner, Sven, 8406 Winterthur (CH); Buni, Richard, 8442 Hettlingen (CH)
(74) Vertreter: Manitz, Finsterwald & Partner GbR

(56) Entgegenhaltungen:
- US-A- 4 474 177
- US-A- 4 944 760
- US-A- 5 431 656

## Beschreibung

Die Erfindung handelt von einem Instrumentenbaukasten für Kniegelenkprothesen mit einem provisorisch verankerbaren Schaft, der am distalen Ende eines Femurknochens für das Einbringen in eine Bohrung in der Richtung der anatomischen Achse vorgesehen ist.

In der US-A-4,759,350 ist ein Instrumentenbaukasten für Kniegelenkprothesen gezeigt, der eine Bohrung, die in der Richtung der anatomischen Achse in die Markraumhöhle gelegt ist, als Ausrichtehilfe für eine Instrumentenanordnung verwendet, bei der die Lage der mechanischen Achse durch die dazu senkrechte transversale Berührungsebene der Femurkondylen festgelegt wird. Dazu wird eine Stange in die Bohrung in der anatomischen Achse eingelegt, an deren vorstehendem Stumpf eine Hilfsplatte soweit schwenkbar ist, dass sie an beiden Femurkondylen anliegt, um sie anschliessend mit Nägeln für einen ersten Resektionsschnitt an der anterioren Seite der Femurkondylen an den Femurkondylen in abgewinkelter Stellung zu befestigen. Anschliessend wird eine zweite Hilfsplatte auf der eben geschnittenen Resektionsfläche aufgesetzt und mittels steckbaren Bolzen zur ersten Hilfsplatte ausgerichtet und durch Nägel auf dieser Resektionsfläche fixiert, um einen stirnseitigen Resektionsschnitt anzubringen. Für die weiteren Lehren und Schnittblöcke werden jeweils die vorher geschnittenen Resektionsflächen als Referenzflächen und Anschläge verwendet. Ein solcher Instrumentensatz setzt ein äusserst exaktes Arbeiten des Orthopäden voraus, da immer wieder Werkzeuge gewechselt und an vorher geschnittenen Resektionsflächen ausgerichtet werden. Noch schwieriger wird die Situation, wenn schon Teile der Kondylen - sei es wegen Zerstörung oder sei es, dass es sich um eine Reoperation handelt - fehlen, um die als Ausgangslage notwendige Ausrichtung der mechanischen Achse festzulegen.

Das Dokument US-A-4 474 177 (WHITESIDE LEO A) offenbart einem provisorisch verankerbaren Schaft, der am distalen Ende eines Femurknochens für das Einbringen in eine Bohrung in der Richtung der anatomischen Achse vorgesehen ist, und drehfest Verankert ist. Der Schaft weist ein Referenzanschlag für Instrumente und eine aussenliegende Parallelführung auf, die verdrehfest in der Richtung einer mechanischen Achse angeordnet ist und gegenüber der mit der anatomischen Achse zusammenfallenden Schaftachse um einen Führungswinkel gegen medial geschwenkt ist.

Aufgabe der Erfindung ist es diese Nachteile zu beseitigen. Sie löst diese Aufgabe, indem der Schaft Vorsprünge zur drehfesten Verankerung aufweist, indem der Schaft einen abschliessenden Kragen als Referenzanschlag für Instrumente aufweist und indem der Schaft eine innenliegende Parallelführung für einen vorstehenden Führungsteil eines Kupplungsstückes aufweist, wobei die Parallelführung verdrehfest in der Richtung einer mechanischen Achse angeordnet ist, die gegenüber der mit der anatomischen Achse zusammenfallenden Schaftachse um einen Führungswinkel α gegen medial geschwenkt ist.

Die Erfindung hat den Vorteil, dass ein Schaft, der aufgrund der Röntgenbilder von Femur und Tibia bezüglich seiner Abmessungen und bezüglich der Parallelführung im passenden Führungswinkel a vorbestimmbar ist, im Femurknochen in einem passenden Verdrehwinkel provisorisch befestigbar ist und als Referenz für alle weiteren Eingriffe am Femur dient. Insbesondere kann der endgültige stirnseitige Resektionsschnitt erst nach dem Festlegen der passenden künstlichen Femurkondylen und nach dem Ueberprüfen derer Funktion mit Probierkondylen

in den beiden Endstellungen der Artikulation vorgenommen werden. Vorteilhafte Weiterbildungen der Erfindung ergeben sich aus den abhängigen Ansprüchen 2 bis 10.

Obwohl der Schaft im Femur praktisch vollständig versenkbar ist und somit späteren Manipulationen nirgends im Weg steht, kann sein Drehwinkel gegenüber einer frontalen oder sagittalen Ebene beim Einschlagen mittels einer positionierenden Aufnahme für die Einschlagvorrichtung und einer an dieser angebrachten Winkelanzeige überprüft werden.

Das Kupplungsstück ist anschliessend an das Führungsteil als Winkelstück mit zwei nacheinander um 90 Grad abgebogenen Schenkeln ausgeführt, die ohne Behinderung als Referenzflächen für weitere Instrumente dienen. Um die mit diesen Instrumenten erzeugten Drehmomente abzufangen, hat der Schaft Vorsprünge in Form von Längsrippen. Zusätzlich sind am zweiten Schenkel, der parallel zum Führungsteil verläuft, Längsschlitze angebracht, durch welche Führungsstifte in den Femur einschlagbar sind, um über den grösseren Hebelarm zwischen Parallelführung und Längsschlitzen auch grössere Momente aufzunehmen. Der Kragen vom Schaft dient als Referenzanschlag für Distanzstücke, die zwischen Manipulierkondylen und Kragen einschiebbar sind, um die optimale Lage der späteren Kondylen durch eine kontrollierte Artikulation zu ermitteln. Da der Kragen des Schaftes eine unabhängige Referenz in der mechanischen Achse bildet, bewährt sich diese Anordnung besonders bei Reoperationen, bei denen der Kragen nicht am mehr Femurknochen anliegt, weil bereits früher eine gehörige Resektion vorgenommen wurde. Die definitive Stellung des Führungsteils gegenüber der Parallelführung in Längsrichtung kann mit Nägeln durch Bohrungen vom zweiten Schenkel am Femur fixiert werden, um alle weiteren Resektionsschnitte für die künstlichen Kondylen von der gleichen Basis aus vorzunehmen. Dadurch, dass mit Manipulierkondylen der passenden Grösse die Artikulation an einer Manipulierplattform der Tibia überprüfbar ist bevor definitive Resektionsschnitte gemacht werden, steigt die Sicherheit für den Operateur. Gleichzeitig wird mit einem universellen in der Richtung der mechanischen Achse verschiebbaren Kupplungsstück die Operationszeit eher kürzer. Für die Führungswinkel α sind Winkel vorgesehen, die beispielsweise einem Valguswinkel von 3°, 5° oder 7° entsprechen. Die Zahl der notwendigen Schäfte wird reduziert, wenn sie zweiteilig mit einem Verschluss ausgeführt sind, um unterschiedliche Schaftlängen mit unterschiedlichen Führungswinkeln α zu kombinieren. Entscheidend für das Setzwerkzeug zum Einschlagen ist dabei, dass das Setzwerkzeug in einer zur Anordnung der Parallelführung definierten Winkelstellung aufsetzbar ist.

Im folgenden wird die Erfindung anhand von Ausführungsbeispielen beschrieben. Es zeigen:
- Fig. 1: Schematisch eine Explosionszeichnung von einem Schaft mit Kupplungsstück für einen Femur und einer am Kupplungsstück befestigbaren Manipulierkondyle, wobei ein steckbares Distanzstück den Abstand zwischen Manipulierkondyle und Schaft festlegt;
- Fig. 2: schematisch eine Explosionszeichnung einer zu Figur 1 passenden Manipulierplattform einer Tibiaprothese;
- Fig. 3: schematisch eine Seitenansicht der Manipulierkondylen von Figur 1 und Figur 2 in abgewinkelter Stellung;
- Fig. 4: schematisch eine Anordnung gemäss Figur 3, bei der jedoch die femurseitige Manipulierkondyle um die Dicke eines Distanzstückes in der Richtung der mechanischen Achse verschoben ist;
- Fig. 5: schematisch eine Einschlagvorrichtung mit einem im Femur eingeschlagenen Schaft;
- Fig. 6: schematisch den eingeschlagenen Schaft von Figur 5 und ein zum Einführen bereites Kupplungsstück; und
- Fig. 7: schematisch ein Kupplungsstück gemäss Figur 1 und Figur 6 mit daran befestigbaren Schnittblöcken.

In den Figuren ist ein Instrumentenbaukasten für Kniegelenkprothesen gezeigt. Ein provisorisch, aber drehfest in der anatomischen Achse 4 des distalen Femurknochens 2 verankerbarer Schaft 1 schliesst zum Gelenk hin mit einem Kragen 6 ab. Vom Kragen 6 gegen das Innere des Schaftes 1 ist eine Parallelführung 7 in der Richtung einer mechanischen Achse 9 angebracht, die um einen Führungswinkel α von der mit der anatomischen Achse 4 zusammenfallenden Schaftachse nach medial wegsteht. Ein Kupplungsstück 10 mit einem vorstehenden Führungsteil 8, welches in der Parallelführung 7 verschiebbar gelagert ist, dient als Aufnahme für Manipulierkondylen 23, um die optimale Lage gegenüber einer Tibiaplattform in den Endlagen der Artikulation zu überprüfen und festzulegen, bevor Schnittblöcke mit dem fixierten Kupplungsstück 10 für endgültige Resektionsschnitte positioniert werden. Der Kragen 6 als Referenzanschlag 30 ist bei Reoperationen von besonderem Vorteil.

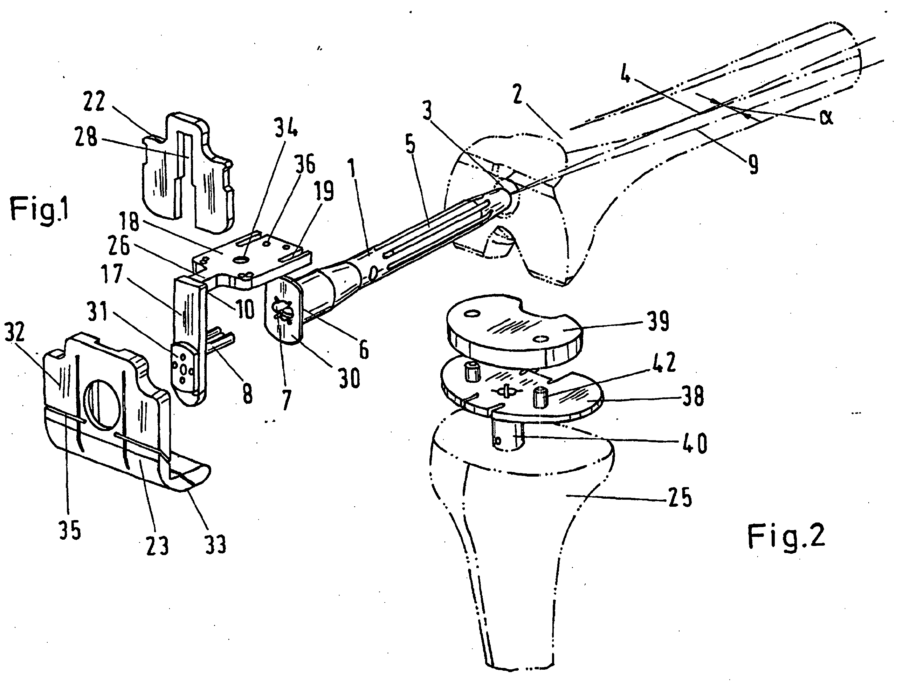

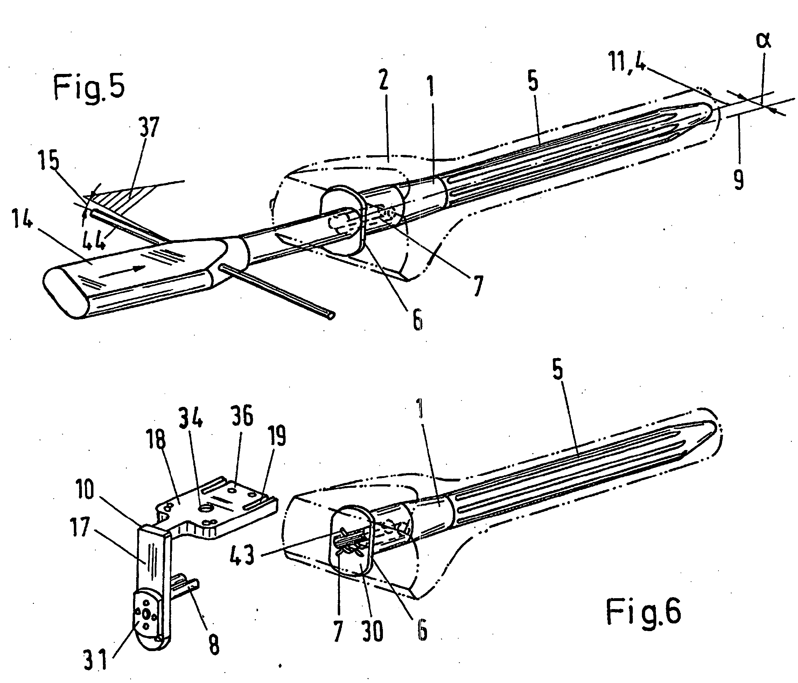

In Figur 1 ist ein Femurknochen 2 mit einer Bohrung 3 in der Richtung seiner Markraumhöhle mit einer anatomischen Achse 4 gezeigt, deren Winkel α zur mechanischen Achse 9 des Femurs bereits bei einer operationsplanung z.B. mittels Röntgenbildern vorbestimmbar ist. Ein zur Bohrung 3 passender Schaft 1 mit einer nach innen gerichteten Parallelführung 7, die im Winkel α gegen medial von der Schaftachse 11 wegsteht, wird mit einer Einschlagvorrichtung 14 eingetrieben und verankert sich drehfest mit Vorsprüngen 5 in Form von Längsrippen im Femur (siehe Fig. 5). Ein Kupplungsstück 10 besitzt einen vorstehenden Führungsteil 8 bestehend aus zwei Stiften, die mit einem Quersteg verbunden sind und in der Parallelführung 7 hin- und hergleiten können. Die Parallelführung 7 besteht aus zwei Bohrungen, die zu einem Langloch verbunden sind, wobei in dem Verbindungsstück Bruchstücke von einer mittleren Bohrung mit Gewindeteilen stehen geblieben sind, um Befestigungsschrauben aufnehmen zu können. Der im Femur versenkbare Schaft schliesst mit einem Kragen 6 ab, dessen Stirnfläche als Anschlag 30 und Referenz in der mechanischen Achse für verschiedene Instrumente dient.

Es versteht sich, dass Schäfte mit unterschiedlichem Winkel a, mit unterschiedlichen Durchmessern und unterschiedlichen Längen zu Verfügung stehen, um ein bestimmtes Spektrum an Fällen abzudecken.

Das Kupplungsstück 10 besitzt im rechten Winkel zum Führungsteil 8 einen ersten Schenkel 17 mit einer Befestigung 31 für weitere Instrumente. Ein zweiter Schenkel 18, der parallel zum Führungsteil 8 in einem Abstand proximal über den Femur 2 ragt, dient als Hilfsführung mit Führungsschlitzen 19 und hat Befestigungen in Form von Gewindelöchern 34 und Bohrungen 36 für weitere Instrumente. Der zweite Schenkel 18 mündet in einem schmalen Joch 26 in den ersten Schenkel 17, um zwischen erstem Schenkel 17 und Anschlag 30 Distanzstücke 22 über das Joch 26 einzustecken, wobei die Distanzstücke 22 über einen Schlitz 28 am Joch 26 geführt sind.

Eine Manipulierkondyle 23, die in diesem Fall auch noch Führungsschlitze 35 für eventuelle Sägeschnitte aufweist, ist am ersten Schenkel 17 stirnseitig befestigbar, wobei der Schenkel 17 soweit in die Kondyle 23 eintaucht, dass beide zum Anschlag 30 hin bündig sind und ein mit der Kondyle 23 und einem bestimmten Distanzstück 22 ermittelter Abstand zum Anschlag 30 auch ohne Kondyle reproduzierbar ist. Die Manipulierkondyle 23 besitzt eine Stirnfläche 32 und eine posteriore Fläche 33, die der Lage der eigentlichen künstlichen Kondylen entsprechen.

In Figur 2 ist eine Manipulierplattform 38 gezeigt, die mit einem Zapfen 40 in der Tibia 25 verankerbar ist. Verschieden hohe Lager mit Lagerfläche 39 sind an Stiften 42 der Manipulierplattform 38 aufsteckbar.

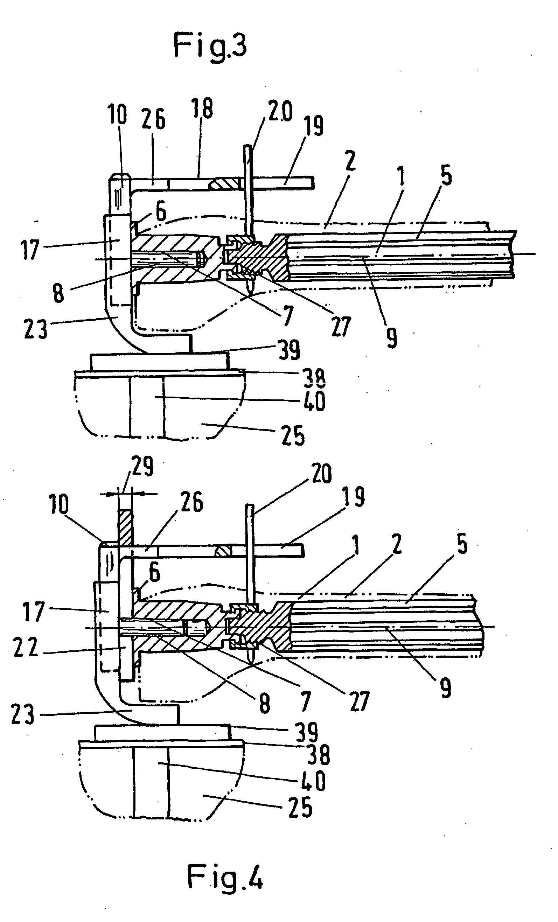

In den Figuren 3 und 4 ist das Zusammenwirken der Manipulierkondylen 23 mit der Manipulierplattform 38 gezeigt. In Figur 3 ist der Schaft 1 im Femur 2 eingeschlagen und mit seinen Rippen 5 gegen Drehung provisorisch verankert. Der Schaft selbst ist zweiteilig aufgebaut. Ein Verankerungszapfen in einer passenden Länge ist über einen Verschluss 27 mit dem Teil der Parallelführung 7 starr verbunden. Das aufgesteckte Kupplungsstück 10 mit Schenkel 17 und die Manipulierkondyle 23 liegen bündig am Kragen 6 an. Mit Hilfe von nicht gezeigten Seitenbändern lässt sich die Lage von Femur 2 und Tibia 25 und die Bänderspannung in den beiden Endlagen der Artikulation überprüfen und femurseitig durch das Einstecken von Distanzstücken 22 unterschiedlicher Dicke 29 (siehe Figur 4) und tibiaseitig durch unterschiedliche Lagerhöhen variieren bis eine optimale Lage gefunden ist. Zur sicheren Führung des Kupplungsstückes 10 sind Stützelemente 20 in Form von Nägeln durch die Führungsschlitze 19 im Femur 2 eingeschlagen und geben seitlich zusätzlich Halt. Wenn die optimale Lage des Kupplungsstückes 10 in der Richtung der mechanischen Achse 9, die sich in der Seitenansicht mit der Schaftachse überdeckt, ermittelt ist, wird der zweite Schenkel 18 mit weiteren Nägeln durch Bohrungen 36 fixiert. Manipulierkondylen 23 und Distanzstücke 22 können entfernt werden, um mit Schnittblöcken die zu dem Distanzstück und der Kondylengrösse passenden Resektionsschnitte vorzunehmen.

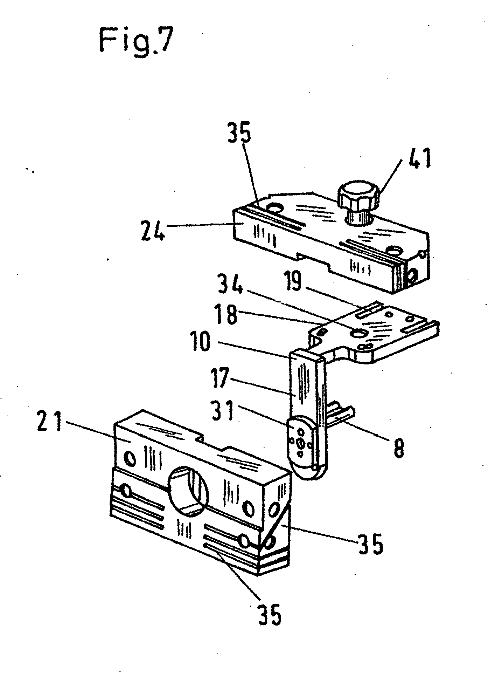

In Figur 7 ist ein vorderer Schnittblock 21, der mit Führungsschlitzen 35 versehen ist, analog zur Manipulierkondyle mit der Befestigung 31 am ersten Schenkel 17 befestigbar. Ein weiterer Schnittblock 24 ist mit einer Befestigungsschraube 41 in ausgerichteter Lage auf dem zweiten Schenkel 18 befestigbar, um eine endgültige vordere Resektionsfläche mit einem einsteckbaren Sägeblatt zu schneiden, welche der Stufung von künstlichen Kondylen und deren zugehörigen Distanzstücken entspricht.

Anhand von Figur 5 und 6 wird das Einschlagen des Schaftes 1 und der Wechsel zum Kupplungsstück 10 beschrieben. Eine in der anatomischen Achse 4 liegende Bohrung wurde bereits vorbereitet. Der Femur 2 besitzt Resektionsflächen von einer früheren Prothese, die aber nicht mehr verwendbar sind. Der Femur selbst liegt mit seiner anatomischen Achse in einer Frontalebene 37. Die Einschlagvorrichtung 14 ist über Flügel (nicht gezeigt) die in Schlitze 43 am Kragen 6 eingreifen, so mit dem Schaft gekoppelt, dass die Schaftachse 11 und die Achse der Parallelführung 7 in einer gleichen und eine Winkelanzeige 44 in Form eines vorstehenden Stabes in einer parallelen Ebene liegen. Beim Einschlagen zentriert sich die Schaftachse 11 von selber in der anatomischen Achse 4. Der Operateur muss lediglich darauf achten, dass die Winkelanzeige 44 parallel zu der Frontalebene 37 bleibt, damit ein Drehwinkel 15 möglichst bei 0 Grad gehalten wird. Auf diese Weise wird die Parallelführung 7 in ausreichender Genauigkeit in der mechanischen Achse 9 des Femurs 2 angebracht, da der Führungswinkel α zwischen Schaftachse 11 und Parallelführung 7 entsprechend dem vorher ermittelten Winkel zwischen anatomischer Achse 4 und mechanischer Achse 9 des Femurs gewählt wurde. Eine andere Möglichkeit, die Zielgenauigkeit beim Einschlagen zu verbessern, besteht darin, auf der Höhe der Epikondylen - also seitlich in der Mitte - jeweils einen stift einzuschlagen und diese stifte zum Ausmitteln des Drehwinkels Null zu verwenden. Das Einschlagen selbst kann mit einem Hammer oder elektrisch angetriebenen Schlagkopf erfolgen. Der Schaft wird in eine vorgeplante Tiefe in den Femur eingeschlagen, wobei die Tiefe mindestens so gross ist, dass die Anschlagfläche 30 vom Kragen 6 für die nachfolgenden Instrumente verwendbar ist. Das aufsteckbare Kupplungsstück 10 ist mit dem Schenkel 17 senkrecht und mit dem Schenkel 18 parallel zum Führungsteil 8 zugeordnet. An beiden Schenkeln sind Anschlagkanten und Befestigungen 31, 34, 36, 19 für nachfolgende Instrumente angeordnet.

Das Prinzip vom provisorisch fixierten Schaft mit einem Kragen als Referenz lässt sich grundsätzlich auch bei Reoperationen auf der Tibiaseite anwenden, wobei jedoch die Parallelführung in der Schaftachse angebracht ist, weil mechanische Achse und Schaftachse bei der Tibia praktisch zusammenfallen.

## Patentansprüche

1. Instrumentenbaukasten für Kniegelenkprothesen mit einem provisorisch verankerbaren Schaft (1), der am distalen Ende eines Femurknochens (2) für das Einbringen in eine Bohrung (3) in der Richtung der anatomischen Achse (4) vorgesehen ist, wobei der Schaft Vorsprünge (5) zur drehfesten Verankerung einen abschliessenden Kragen (6) als Referenzanschlag für Instrumente und eine innenliegende Parallelführung (7) für einen vorstehenden Führungsteil (8) eines Kupplungsstückes (10) aufweist, wobei die Parallelführung (7) verdrehfest in der Richtung einer mechanischen Achse (9) angeordnet ist, die gegenüber der mit der anatomischen Achse (4) zusammenfallenden Schaftachse (11) um einen Führungswinkel a gegen medial geschwenkt ist.

2. Instrumentenbaukasten nach Anspruch 1, **dadurch gekennzeichnet, dass** der Schaft an seiner an den Kragen (6) anschliessenden Stirnseite (12) eine positionierende Aufnahme (13) für eine Einschlagvorrichtung (14) mit einer vergrösserten Anzeige des Drehwinkels (15) des Schaftes (1) gegenüber einer frontalen oder sagittalen Ebene aufweist.

3. Instrumentenbaukasten nach Anspruch 1 oder 2, **dadurch gekennzeichnet, dass** das Kupplungsstück (10) als Winkelstück (16) ausgeführt ist, dessen erster Schenkel (17) senkrecht zum vorstehenden Führungsteil (8) und dessen zweiter Schenkel (18) anterior und parallel zum Führungsteil (8) angeordnet ist, wobei der Parallelabstand so gross gewählt ist, dass der zweite Schenkel (18) keine Berührung mit dazwischenliegenden Femurknochenteilen haben kann.

4. Instrumentenbaukasten nach Anspruch 3, **dadurch gekennzeichnet, dass** der zweite Schenkel mit mindestens einem Führungsschlitz (19) versehen ist, der parallel zum Führungsteil (8) verläuft, um mit dem Führungsschlitz (19) eine Hilfsführung gegen Drehung für ein am Femurknochen befestigbares stützelement (20) zu ermöglichen.

5. Instrumentenbaukasten nach einem der Ansprüche 1 bis 4, **dadurch gekennzeichnet, dass** auf dem Kupplungsstück (10) ein Schnittblock (21, 24) in einer vorgesehenen Ausrichtposition zum Kupplungsstück (10) befestigbar ist, um Resektionsschnitte zwangsläufig in einer vorgesehenen Ausrichtung zur mechanischen Achse (9) zu ermöglichen.

6. Instrumentenbaukasten nach einem der Ansprüche 1 bis 5, **dadurch gekennzeichnet, dass** auf der Stirnseite des ersten Schenkels (17) in einer vorgesehenen Ausrichtposition Manipulierkondylen (23) befestigbar sind, um gegenüber einer Manipulierplattform (38) für die Tibia (25) die Lage der Endstellungen einer Gelenkartikulation kontrollierbar zu machen.

7. Instrumentenbaukasten nach Anspruch 6, **dadurch gekennzeichnet, dass** auf der Rückseite des ersten Schenkels (17) verschieden dicke Distanzstücke an den Manipulierkondylen (23) und am Kragen (6) als Referenzanschlag (30) anliegend einsteckbar sind, um in der Führungsrichtung der mechanischen Achse (9) die optimale Distanz für die späteren künstlichen Kondylen zu ermitteln.

8. Instrumentenbaukasten nach Anspruch 6, **dadurch gekennzeichnet, dass** verschieden grosse künstliche Kondylen und diesen entsprechend verschieden grosse Manipulierkondylen (23) vorgesehen sind.

9. Instrumentenbaukasten nach einem der Ansprüche 1 bis 8, **dadurch gekennzeichnet, dass** mehrere Schäfte vorhanden sind, die sich in ihrer Länge und in der Grösse vom Führungswinkel α zwischen Schaftachse (11) und Führungsrichtung respektive zwischen anatomischer Achse (4) und mechanischer Achse (9) unterscheiden.

10. Instrumentenbaukasten nach Anspruch 9, **dadurch gekennzeichnet, dass** die Schäfte zweiteilig mit Verschluss (27) ausgeführt sind, um verschiedene schaftlängen und Führungen mit verschiedenen Führungswinkeln α zu kombinieren.

## Claims

1. Modular instrument system for knee joint prostheses comprising a provisionally anchorable shaft (1) which is provided at the distal end of a femur bone (2) for the introduction into a bore (3) in the direction of the anatomical axis (4) wherein the shaft has projections (5) for a rotationally fixed anchoring, a terminating collar (6) as a reference abutment for instruments, and an inwardly disposed parallel guide (7) for a projecting guide part (8) of a coupling piece (10), with the parallel guide (7) being arranged rotationally fixedly in the direction of a mechanical axis (9) which is pivoted towards medial by a guide angle α with respect to the shaft axis (11) coinciding with the anatomical axis (4).

2. Modular instrument system in accordance with claim 1 **characterised in that** the shaft has at its front surface (12), which adjoins the collar (6), a positioning receiver or seat (13) for a drive-in device (14) with an enlarged indication of the angle of rotation (15) of the shaft (1) with respect to a frontal or sagittal plane.

3. Modular instrument system in accordance with claim 1 or claim 2 **characterised in that** the coupling piece (10) is formed as an angled piece (16) of which the first limb (17) is arranged perpendicular to the projecting guide part (8) and of which the second limb (18) is arranged to anterior and parallel to the guide part (8), with the parallel spacing being chosen to be so large that the second limb (18) can have no contact with intervening femur bone parts.

4. Modular instrument system in accordance with claim 3 **characterised in that** the second limb is provided with at least one guide slot (19) which extends parallel to the guide part (8) in order to enable an auxiliary guiding against rotation with the guide slot (19) for a support element (20) which can be mounted on the femur bone.

5. Modular instrument system in accordance with any one of the claims 1 to 4 **characterised in that** a resection block (21, 24) can be mounted on the coupling piece (10) in a predetermined orientation position with respect to the coupling piece (10) in order to enable resections in a compulsory manner in a predetermined orientation with respect to the mechanical axis (9).

6. Modular instrument system in accordance with any one of the claims 1 to 5 **characterised in that** manipulation condyles (23) can be fastened to the end face of the first limb (17) in a predetermined orientation in order to make the location of the final positions of a joint articulation checkable with respect to a manipulation platform (38) for the tibia (25).

7. Modular instrument system in accordance with claim 6 **characterised in that**, on the reverse side of the first limb (17), spacers of different thicknesses can be inserted to lie in contact at the manipulation condyles (23) and at the collar (6) as reference abutments (30) in order to determine the ideal distance in the guide direction of the mechanical axis (9) for the later artificial condyles.

8. Modular instrument system in accordance with claim 6 **characterised in that** artificial condyles of different sizes and manipulation condyles (23) of different sizes corresponding to them are provided.

9. Modular instrument system in accordance with any one of the claims 1 to 8 **characterised in that** a plurality of shafts are present which differ in their lengths and in the size of the guide angle α between the shaft axis (11) and the guide direction or, respectively, between the anatomical axis (4) and the mechanical axis (9).

10. Modular instrument system in accordance with claim 9 **characterised in that** the shafts are made in two parts with a lock (27) in order to combine a plurality of shaft lengths and guides with different guide angles α.

## Revendications

1. Système modulaire d'instruments pour des prothèses de l'articulation du genou, comportant une tige (1) susceptible d'être ancrée provisoirement et prévue à l'extrémité distale d'un os de fémur (2) pour l'introduction dans un perçage (3) dans la direction de l'axe anatomique (4), dans lequel la tige présente des saillies (5) pour l'ancrage solidaire en rotation, une collerette (6) terminale à titre de butée de référence pour des instruments, et un guidage parallèle interne (7) pour une partie de guidage saillante (8) d'un élément d'accouplement (10), le guidage parallèle (7) étant agencé solidairement en rotation dans la direction d'un axe mécanique (9) qui est pivoté en direction médiale d'un angle de guidage α par rapport à l'axe de tige (11) coïncidant avec l'axe anatomique (4).

2. Système modulaire d'instruments selon la revendication 1, **caractérisé en ce que** la tige présente sur son côté frontal (12) raccordé à la collerette (6) un évidement de positionnement (13) pour un dispositif de frappe (14) comprenant un affichage agrandi de l'angle de rotation (15) de la tige (1) par rapport à un plan frontal ou sagittal.

3. Système modulaire d'instruments selon l'une ou l'autre des revendications 1 et 2, **caractérisé en ce que** l'élément d'accouplement (10) est réalisé sous la forme d'une pièce en équerre (16) dont la première branche (17) s'étend perpendiculairement à la partie de guidage saillante (8) et dont la seconde branche (18) est agencée en position antérieure et parallèlement à la partie de guidage (8), la distance parallèle étant choisie d'une taille telle que la seconde branche (18) ne peut pas être en contact physique avec des parties interposées de l'os de fémur.

4. Système modulaire d'instruments selon la revendication 3, **caractérisé en ce que** la seconde branche est pourvue d'au moins une fente de guidage (19) qui s'étend parallèlement à la partie de guidage (8) afin de permettre, conjointement avec la fente de guidage (19), un guidage auxiliaire à l'encontre d'une rotation pour un élément de soutien (20) susceptible d'être fixé sur l'os de fémur.

5. Système modulaire d'instruments selon l'une des revendications 1 à 4, **caractérisé en ce que** sur l'élément d'accouplement (10) peut venir se fixer un bloc de coupe (21, 24) dans une position d'orientation prédéterminée par rapport à l'élément d'accouplement (10), afin de permettre de pratiquer des coupes de résection obligatoirement dans une orientation prédéterminée par rapport à l'axe mécanique (9).

6. Système modulaire d'instruments selon l'une des revendications 1 à 5, **caractérisé en ce que** sur le côté frontal de la première branche (17) peuvent venir se fixer des condyles de manipulation (23) dans une position d'orientation prédéterminée, afin de rendre contrôlable la situation des positions terminales d'une articulation par rapport à une plate-forme de manipulation (38) pour le tibia (25).

7. Système modulaire d'instruments selon la revendication 6, **caractérisé en ce que** sur le côté arrière de la première branche (17), des éléments écarteurs de différentes épaisseurs peuvent être enfichés en prenant appui contre les condyles de manipulation (23) et contre la collerette (6) à titre de butée de référence (30), afin de déterminer dans la direction de guidage de l'axe mécanique (9) la distance optimale pour les condyles artificiels ultérieurs.

8. Système modulaire d'instruments selon la revendication 6, **caractérisé en ce qu'**il est prévu des condyles artificiels de différentes tailles et en correspondance de ceux-ci des condyles de manipulation (23) de différentes tailles.

9. Système modulaire d'instruments selon l'une des revendications 1 à 8, **caractérisé en ce qu'**il existe plusieurs tiges qui se distinguent de par leur longueur et de par la taille de leur angle de guidage α respectivement entre l'axe de tige (11) et la direction de guidage et entre l'axe anatomique (4) et l'axe mécanique (9).

10. Système modulaire d'instruments selon la revendication 9, **caractérisé en ce que** les tiges sont réalisées en deux pièces avec un obturateur (27) afin de combiner différentes longueurs de tige et différents guidages avec différents angles de guidage α.
